# EUROPÄISCHE PATENTSCHRIFT

(11) **EP 1 088 518 B1**
(45) Veröffentlichungstag und Bekanntmachung des Hinweises auf die Patenterteilung: **29.06.2005**
(21) Anmeldenummer: 00810749.2
(22) Anmeldetag: 23.08.2000
(51) Int. Cl.: A61B 17/064

(54) **Gewebehalter**
Surgical fixing device
Dispositif chirurgical de fixation

(30) Priorität: 29.09.1999 EP 99810880
(43) Veröffentlichungstag der Anmeldung: 04.04.2001
(73) Patentinhaber: Zimmer GmbH, 8404 Winterthur (CH)
(72) Erfinder: Jakob, Roland Peter, Prof. Dr. Med., 1787 Môtier (CH); Müller, Werner, Dr., 8542 Wiesendangen (CH); Frei, Heribert, 8404 Winterthur (CH)
(74) Vertreter: Manitz, Finsterwald & Partner Gbr

(56) Entgegenhaltungen:
- EP-A- 0 913 123
- WO-A-99/01071
- FR-A- 2 719 993
- US-A- 5 474 557
- US-A- 5 810 851
- US-A- 5 957 940

## Beschreibung

Die Erfindung betrifft einen Gewebehalter gemäss dem Oberbegriff des unabhängigen Patentanspruchs.

Bei Läsionen von Gewebe möchte man im Normalfall einen möglichst guten und schnellen Heilungsprozess erreichen. Dies gilt insbesondere auch für Läsionen des Meniskus des Knies, speziell für kleine Risse im Meniskus. Solche Meniskusläsionen heilen beim erwachsenen Menschen nicht spontan, sondern die Gewebeteile müssen zu diesem Zweck gegeneinander gedrückt und fixiert werden. Nur eine ausreichendes Zusammendrücken der Gewebeteile gegeneinander erlaubt einen Heilungsprozess. Angestrebt wird nach dem Heilen eine möglichst glatte Oberfläche des Meniskus ohne Kratzer etc,. weil auf dieser Meniskusoberfläche ja die Femurkondylen gleiten und die Artikulationsflächen der Kondylen knorpelig und daher sehr empfindlich für Kratzer in der Meniskusoberfläche sind.

Um eine ausreichendes Zusammendrücken der Gewebeteile gegeneinander zu bewirken und die Gewebeteile dann in dieser Position zu halten, haben sich verschiedene Behandlungstechniken etabliert. Eine solche Behandlungstechnik ist das Fixieren der Gewebeteile durch Nähen, wobei unterschiedlichste Nähtechniken zum Einsatz kommen. Das Nähen eines Meniskusrisses und ein hierfür geeignetes Instrument ist beispielsweise in der WO-A-98/31288 beschrieben. Diese Art der Fixierung (Nähen) der Gewebeteile ist zwar zuverlässig, aber von der Operationstechnik her ziemlich aufwendig, erst recht dann, wenn der Eingriff arthroskopisch erfolgt (Zugänglichkeit), was in solchen Fällen mehr und mehr der Fall ist und wohl schon den Regelfall darstellt.

Eine weitere Behandlungstechnik ist das Fixieren der Gewebeteile durch Einbringen eines Implantats. Als Implantate werden dabei beispielsweise Stifte mit Widerhaken am Stiftkörper (erste Befestigungsmittel) und mit einem Vorsprung (zweite Befestigungsmittel) am stumpfen Ende des Stiftköroers verwendet, wie dies beispielsweise in der WO-A-97118761 sowie EP-A-913123 beschrieben ist.

Die zweiteilge Form des Anspruchs 1 basiert sich auf diesem letzten Dokument.

Die Widerhaken verhaken sich nach dem Einbringen in dem Gewebeteil jenseits des Risses, ziehen dieses Gewebeteil in Richtung auf den Vorsprung am stumpfen Ende des Stifts in dem diesseitigen Teil des Risses und halten die beiden Gewebeteile in dieser Position, sodass der Riss heilen kann. Andere Implantate, welche bei der Fixierung der Gewebeteile verwendet werden, sind beispielsweise Schrauben, welche zwei Abschnitte aufweisen, die jeweils mit einem Gewinde versehen sind. Die beiden Gewinde (erste und zweite Befestigungsmittel) weisen jedoch eine unterschiedliche Steigung auf (siehe z.B. US-A-5,569,252). Durch diese unterschiedliche Steigung der Gewinde wird dann, wenn das eine Gewinde jenseits des Risses und das andere Gewinde diesseits des Risses im Meniskus greifen, die beiden Gewebeteile zusammengezogen und in dieser Position gehalten, sodass der Riss heilen kann.

Bei diesen Arten des Fixierens der Gewebeteile mit Hilfe von Implantaten wird das Implantat (der Stift mit den Widerhaken bzw. die Schraube) in Bezug auf die Dicke des Meniskus jeweils zentral, d.h. ungefähr in die Mitte, eingebracht. Wichtig ist jedenfalls, dass von dem Implantat absolut nichts in die Meniskusoberfläche vorsteht, denn auf dieser müssen ja die Femurkondylen gleiten. Diese Arten der Fixierung (mittels Implantaten) sind von der Operationstechnik her einfacher als die Nähtechniken. Mit zunehmendem Alter der Patienten wird es aber schwieriger, diese Art der Fixierung einzusetzen, weil der innere Teil ("Kern") des Meniskus zunehmend mukoid wird, also weich, sodass die Implantate im Innern ("Kern") des Meniskus nur noch schlecht fixiert werden können. Es wird dann vermehrt auf die bereits beschriebenen, von der Operationstechnik her jedoch vergleichsweise aufwendigen, Nähtechniken ausgewichen, weil die zentrale Verankerung des Implantats (im "Kern" des Meniskus) nicht mehr in Frage kommt.

Es ist daher eine Aufgabe der Erfindung, einen Gewebehalter (Implantat) vorzuschlagen, welcher einfach an den Gewebeteilen befestigbar ist und die Gewebeteile in einer gewünschten Position relativ zueinander hält. Insbesondere soll dieser Gewebehalter für die Behandlung von Rissen im Meniskus geeignet sein und zwar auch für Patienten, bei denen der innere Teil ("Kern") des Meniskus schon weich ist, sodass man sonst auf Nähtechniken ausweichen würde.

Diese Aufgabe wird erfindungsgemäss durch einen Gewebehalter gelöst, wie er durch die Merkmale des unabhängigen Patentanspruchs charakterisiert ist. Besonders vorteilhafte Ausgestaltungen des erfindungsgemässen Gewebehalters ergeben sich aus den abhängigen Patentansprüchen.

Insbesondere umfassen die ersten und zweiten Befestigungsmittel des Gewebehalters (die Befestigungsmittel diesseits und jenseits des Risses) jeweils mindestens einen gekrümmten Dorn, welcher sich um die Längsachse des Gewebehalters herum erstreckt. Selbst wenn also der Gewebehalter zentral, also in den weichen "Kern" des Meniskus eingebracht wird, erfolgt die Verbindung mit den beiden Gewebeteilen mit Hilfe des gekrümmten Dorns, der sich um die Längsachse des Gewebehalters herum erstreckt, also nicht im "Kern" des Meniskus, sondern in einem Bereich unterhalb der Oberfläche des Meniskus, wo der Meniskus noch genügend Festigkeit aufweist, um eine ausreichend gute Fixierung des Gewebehalters zu erreichen. Der Gewebehalter kann also - gesamthaft betrachtet - durchaus in den möglicherweise weichen "Kern" des Meniskus eingebracht werden, weil die Verbindung mit dem Gewebe eben in einem Bereich erfolgt (unterhalb der Oberfläche des Meniskus), wo das Gewebe eine ausreichende Festigkeit aufweist, um eine gute Fixierung des Gewebehalters zu erreichen.

Bei einem vorteilhaften Ausführungsbeispiel umfassen die Mittel, welche die Dorne und damit die Gewebeteile relativ zueinander halten, einen in Richtung der Längsachse des Gewebehalters verlaufenden Verbindungssteg. Die Dorne erstrecken sich dabei um diesen Verbindungssteg herum. Dies ist eine von der konstruktiven Ausgestaltung und vom Handling her einigermassen einfache und funktionstüchtige Ausführungsvariante des Gewebehalters.

Bei einer vorteilhaften Weiterbildung nimmt der axiale Abstand zwischen dem Dorn bzw. den Dornen der ersten Befestigungsmittel und dem Dorn bzw. den Dornen der zweiten Befestigungsmittel in Richtung zum freien Ende der Dorne hin zu, also vom freien Ende der Dorne ausgehend zum Verbindungssteg hin nimmt dieser Abstand ab. Dies bewirkt, dass beim Eindrehen des Gewebehalters die Gewebeteile zusammengezogen werden können (wie dies bei der Behandlung eines Risses im Meniskus erwünscht ist), dass also der Gewebehalter als Klammer wirkt.

Bei einem vorteilhaften Ausführungsbeispiel erstrecken sich die Dorne wendelförmig um die Längsachse des Gewebehalters bzw. um den Verbindungssteg herum. Durch diesen wendelförmigen Verlauf kann man erreichen, dass beim Eindrehen des Gewebehalters die beiden Gewebeteile zusammengezogen werden und der Gewebehalter als Klammer wirkt. Grundsätzlich ist es jedoch auch möglich (nämlich dann, wenn der Abstand zwischen dem Dorn bzw. den Dornen der ersten Befestigungsmittel und dem Dorn bzw. den Dornen der zweiten Befestigungsmittel in Richtung zum freien Ende der Dorne hin abnimmt) die Gewebeteile auseinanderzuziehen, sofern die erwünscht ist. Bei der vorgenannten Behandlung eines Risses im Meniskus trifft dies allerdings nicht zu.

Bei einer Weiterbildung dieses Gewebehalters nimmt die axiale Dicke eines Dorns in Richtung zum freien Ende des Dorns hin ab. Dadurch kann man die Wendelform erreichen und bewirken, dass beim Eindrehen des Gewebehalters die beiden Gewebeteile zusammengezogen werden und der Gewebehalter als Klammer wirkt. Je nachdem, auf welche Weise die axiale Dicke abnimmt, kann man auch erreichen, dass beim Eindrehen des Gewebehalters die Gewebeteile auseinandergezogen werden, sofern dies erwünscht ist.

Bei einer anderen Weiterbildung sind die Dorne im wesentlichen zylindrisch ausgebildet, aber dennoch verlaufen sie wendelförmig. Am freien Ende können sich die Dorne natürlich verjüngen, um das Eindringen in das jeweilige Gewebeteil zu erleichtern. Auch mit dieser Weiterbildung kann entweder die Funktion einer Klammer bewirkt werden oder die Gewebeteile können auseinander gezogen werden, je nachdem, wie die Wendel verläuft.

Bei einem weiteren Ausführungsbeispiel des Gewebehalters umfassen die ersten und zweiten Befestigungsmittel jeweils zwei Dorne, wobei die Dorne des jeweiligen Befestigungsmittels an gegenüberliegenden Orten von dem Verbindungssteg ausgehen und sich dann in gleicher Richtung um den Verbindungssteg herum erstrecken. Dies hat den Vorteil, dass beim Einbringen des Gewebhalters der Gewebehalter selbst in ungünstigen Fällen nicht sehr weit gedreht werden muss, bis ein Dorn in das jeweilige Gewebeteil einsticht und somit mit dem jeweiligen Gewebeteil verbunden ist.

Bei einem weiteren vorteilhaften Ausführungsbeispiel ist am Gewebehalter ein Ansatzstück für ein Werkzeug vorgesehen, mit welchem eine Verbindung des Gewebehalters an den Gewebeteilen bewirkt werden kann. Dieses Ansatzstück kann beispielsweise ein Aufnahmeschlitz für ein Drehwerkzeug (nach Art eine Schraubendrehers) aufweisen oder aber auch anders ausgebildet sein, um einem Werkzeug die Bewegung des Gewebehalters zu ermöglichen.

Schliesslich ist es vorteilhaft, wenn der Gewebehalter aus einem bioresorbierbaren Material, insbesondere aus Polylactiden, hergestellt ist. Dadurch erfolgt einerseits die gewünschte Fixierung und Heilung, andererseits muss der Gewebehalter nicht noch einmal durch einen operativen Eingrif entfernt werden.

Im folgenden wird die Erfindung anhand der Zeichnung näher erläutert. Dabei zeigen in schematischer Darstellung und/oder teilweise im Schnitt:
- Fig. 1: eine perspektivische Ansicht eines Ausführungsbeispiels eines erfindungsgemässen Gewebehalters,
- Fig. 2: eine Seitenansicht des Gewebehalters nach Fig. 1,
- Fig. 3: eine Stirnansicht des Gewebehalters nach Fig. 1 aus der Richtung des Ansatzstücks,
- Fig. 4: eine schematische Darstellung zur Erläuterung des Einbringens des Gewebehalters bei der Behandlung eines Risses im Innenmeniskus (meniscus medialis)
und
- Fig. 5: ein weiteres Ausführungsbeispiel eines erfindungsgemässen Gewebehalters in Seitenansicht.

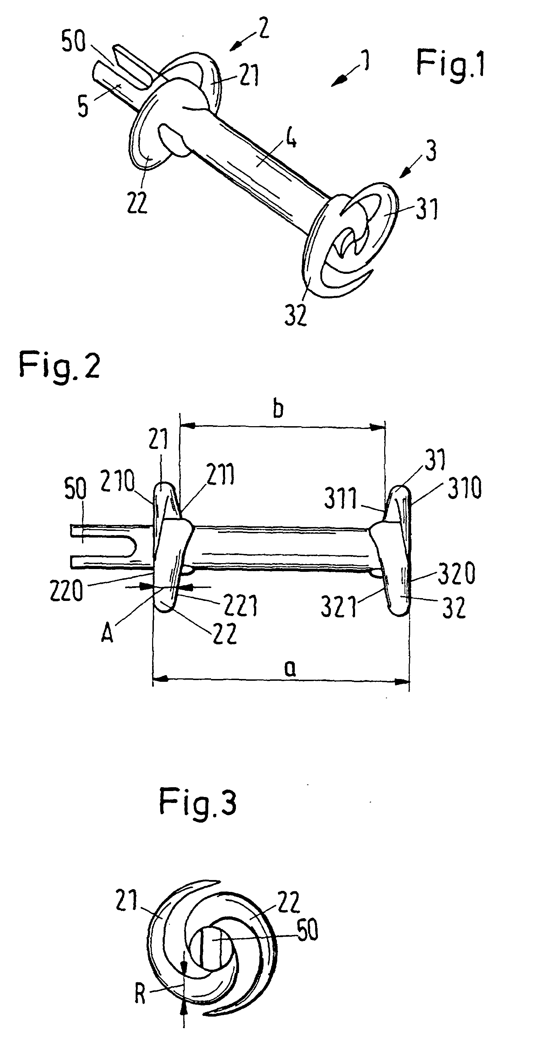

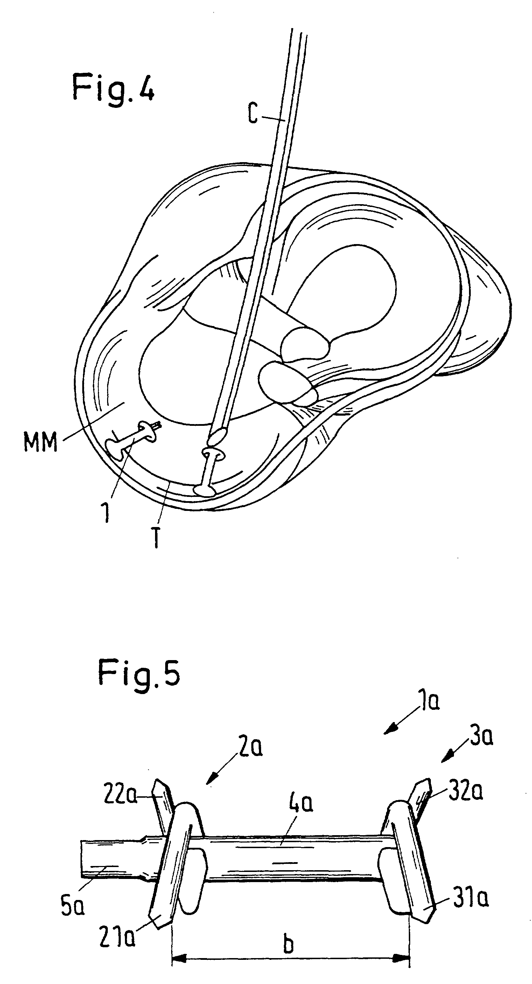

In Fig. 1 erkennt man ein Ausführungsbeispiel eines erfindungsgemässen Gewebehalters 1. Der erfindungsgemässe Gewebehalter 1 weist erste Befestigungsmittel 2 in Form der zwei Dorne 21 und 22 auf sowie zweite Befestigungsmittel 3 in Form der zwei Dorne 31 und 32, welche mit den Gewebeteilen diesseits und jenseits eines Risses im Meniskus verbindbar sind (siehe Fig. 4). Zwischen den ersten Befestigungsmitteln 2 und den zweiten Befestigungsmitteln 3 erstreckt sich ein Verbindungssteg 4. Ferner erkennt man in Fig. 1, dass der Gewebehalter noch ein Ansatzstück 5 aufweist für ein Werkzeug (nicht dargestellt), mit dessen Hilfe der Gewebehalter 1 mit den Gewebeteilen (z.B. mit den Meniskusteilen diesseits und jenseits des Risses, siehe Fig. 4) verbunden werden kann. Das Ansatzstück 5 kann z.B. mit einem Schlitz 50 versehen sein, wie in Fig. 1 und Fig. 2 gezeigt, in welchen ein Drehwerkzeug nach dem Prinzip eines Schraubendrehers eingreifen kann, es können aber auch andere Mittel vorgesehen sein, mit denen eine Bewegung des Gewebehalters 1 bewirkt werden kann.

Aus Fig. 1 und Fig. 2 erkennt man, dass die Dorne 21 und 22 der ersten Befestigungsmittel 2 bzw. die Dorne 31 und 32 der zweiten Befestigungsmittel 3 gekrümmt sind und sich jeweils ausgehend von dem Verbindungssteg 4 um den Verbindungssteg 4 herum erstrecken. In Fig. 3 erkennt man, dass sich die Dorne 21 und 22 (entsprechendes gilt für die Dorne 31 und 32) wendelförmig um den Verbindungssteg 4 herum erstrecken. Grundsätzlich würde es ausreichen, wenn die ersten Befestigungsmittel 2 und die zweiten Befestigungsmittel 3 jeweils nur einen Dorn aufweisen würden, das gezeigte Ausführungsbeispiel mit jeweils zwei Dornen, die an gegenüberliegenden Orten von dem Verbindungssteg 4 ausgehen, also etwa um 180° auf dem Umfang des Verbindungsstegs 4 versetzt sind, ist jedoch insofern vorteilhaft, als der Gewebehalter 1 beim Einbringen nicht erst um einen grossen Winkel gedreht werden muss, sondern schnell in das jeweilige Gewebeteil einsticht. Die beiden Dorne 21 und 22 sowie 31 und 32 erstrecken sich dabei jeweils in der gleichen Richtung um den Verbindungssteg 4 herum und verlaufen wendelförmig.

Dadurch dass sich die Dorne 21 und 22 sowie 31 und 32 um den Verbindungssteg 4 herum erstrecken, kann der Gewebehalter 1 - gesamthaft betrachtet - in den "Kern" des Meniskus eingebracht werden, auch wenn dieser bereits weich ist und keine gute Verankerung für einen derartigen Gewebhalter mehr ermöglichen würde, weil ja die Dorne in einem Bereich unterhalb der Oberfläche des Meniskus in den Meniskus eingreifen, also in einem Bereich, wo der Meniskus eine ausreichende Festigkeit aufweist, um eine gute Verankerung des Gewebehalters zu gewährleisten. Gleichzeitig bleibt die Oberfläche des Meniskus von dem Dorn unbeeinträchtigt, was für ein einwandfreies Zusammenwirken der Artikulationsfläche der jeweiligen Femurkondyle mit dem Meniskus wichtig ist.

In Fig. 3 erkennt man gut, dass die radiale Dicke R der Dorne 21 und 22 (entsprechendes gilt für die Dorne 31 und 32) ausgehend vom Verbindungssteg 4 in Richtung zum freien Ende des jeweiligen Dorns hin abnimmt. Dies begründet einerseits eine stabile Befestigung der Dorne am Verbindungssteg 4, andererseits müssen die Dorne am freien Ende einigermassen spitz sein, damit sie in die Gewebeteile (hier: Meniskusteile) eindringen können. Dabei kann der Dorn am freien Ende - wie in Fig. 3 gezeigt, leicht über die tangentiale Richtung hinweg nach aussen stehen, sodass beim Eindrehen der Dorn in jedem Fall in das betreffende Gewebeteil einsticht und nicht etwa unter dem Gewebeteil hindurchrutschen kann.

Auch die axiale Dicke A der Dorne nimmt ausgehend vom Verbindungssteg 4 in Richtung zum freien Ende des jeweiligen Dorns hin ab, was man in Fig. 2 gut erkennen kann. Dies gilt beim vorliegenden Ausführungsbeispiel sowohl für die Dorne 21 und 22 der ersten Befestigungsmittel 2 als auch für die Dorne 31 und 32 der zweiten Befestigungsmittel 3. Dabei verlaufen die jeweiligen in axialer Richtung nach aussen weisenden Flächen der Dorne 21 und 22 der ersten Befestigungsmittel 2 und die nach aussen weisenden Flächen der Dorne 31 und 32 der zweiten Befestigungsmittel 3 in in einer Ebene senkrecht zur axialen Richtung, also senkrecht zur Längsachse des Verbindungsstegs 4. Der Abstand a zwischen den nach aussen weisenden Flächen 210 bzw. 220 der Dorne 21 und 22 der ersten Befestigungsmittel 2 und den entsprechenden nach aussen weisenden Flächen 310 bzw. 320 Dornen 31 und 32 der zweiten Befestigungsmittel 3 ist also konstant. Der Abstand b zwischen den nach innen weisenden Flächen 211 bzw. 221 der Dorne 21 und 22 der ersten Befestigungsmittel 2 und den entsprechenden nach innen weisenden Flächen 311 bzw. 321 der Dorne 31 und 32 der zweiten Befestigungsmittel 3 nimmt jedoch - vom freien Ende der Dorne ausgehend in Richtung zum Verbindungssteg 4 betrachtet - ab. Dies führt zu der Wendelform und bewirkt, dass beim Einbringen des Gewebehalters 1 die beiden Gewebeteile beim Eindrehen des Gewebehalters 1 zusammengezogen werden und somit die Wirkung einer Klammer erzielen. Im Grunde würde es zur Erzielung dieser Klammerwirkung ausreichen, wenn nur die ersten Befestigungsmittel 2 oder nur die zweiten Befestigungsmitteln 3 Dorne aufweisen würden, deren nach innen weisende Flächen so ausgebildet sind wie oben beschrieben. Dadurch, dass dies aber bei beiden Befestigungsmitteln der Fall ist, wird die Klammerwirkung noch verstärkt.

Es ist leicht einzusehen, dass dann, wenn es erwünscht ist, Gewebeteile auseinanderzuziehen, die Dorne so ausgebildet sein können, dass der Abstand zwischen den nach innen weisenden Flächen der Dorne konstant gehalten wird und der Abstand zwischen den nach aussen weisenden Flächen zunimmt (vom Steg zum freien Ende hin betrachtet), um die Gewebeteile auseinanderzuziehen und sie dann in dieser Position zu halten. Auf diese Weise kann dann eine Spreizwirkung erzielt werden.

Besonders vorteilhaft ist es, wenn der Gewebehalter 1 aus bioresorbierbaren Materialien, insbesondere aus Polylactiden, hergestellt ist. Dann kann nämlich einerseits der Riss zusammengehalten werden, sodass die Fixierung und Heilung erfolgten kann, andererseits muss kein weiterer operativer Eingriff durchgeführt werden, um den Gewebehalter 1 später wieder zu entfernen.

In Fig. 4 ist schematisch angedeutet, wie ein Gewebehalter im Falle eines Risses T im Innenmeniskus MM (meniscus medialis) eingebracht werden kann. Im Bereich des vorderen Endes des Risses T ist bereits ein Gewebehalter 1 eingebracht. Im Bereich des hinteren Endes wird er gerade eingebracht. Hierzu wird zunächst eine Kanüle C durch den Riss T hindurch gestochen, bis sie gerade eben in den anderen Meniskusteil eingedrungen ist. Durch den Innenraum dieser Kanüle C hindurch wird der Gewebhalter 1 dann mit einem Schraubendreher (nicht dargestellt), der in den Schlitz 50 des Ansatzstücks 5 eingreift (siehe Fig. 1 und Fig. 2), bis zum vorderen Ende der Kanüle C geschoben. Der Schraubendreher kann an seinem distalen Ende eine Bohrung aufweisen, welche die eigentliche Klinge des Schraubendrehers umgibt. Diese Bohrung nimmt das Ansatzstück 5 des Gewebehalters auf. Die von der Bohrung umgebene Klinge des Schraubendrehers kann dann in den Schlitz 50 des Ansatzstücks 5 eingreifen, ohne dass die Klinge radial aus dem Schlitz 50 herausgleiten kann, weil sie in der Bohrung gefangen ist, die die Klinge umgibt. Gegen axiales Herausgleiten des Gewebehalters 1 aus der Bohrung des Schraubendrehers reicht die Reibung zwischen Gewebehalter 1 und Schraubendreher aus.

Ist nun die Kanüle C durch den Riss T hindurch gerade eben bis in den anderen Meniskusteil eingedrungen, so wird der Schraubendreher mit dem Gewebehalter 1 in der Kanüle C bis ans distale Ende der Kanüle vorgeschoben. Dann wird die Kanüle C ein kleines Stück zurückgezogen. Anschliessend wird der Gewebehalter 1 mit Hilfe des Schraubendrehers gedreht. Die Dorne 21 bzw. 22 und 31 bzw. 32 dringen dann in das diesseits des Risses T und das jenseits des Risses T angeordnete Gewebeteil ein, und durch ein weiteres Drehen (z.B. maximal 1/4-Drehung) des Gewebehalters werden die Gewebeteile zusammengezogen und auf diese Weise der Riss T geschlossen.

Vor dem Einführen der Kanüle C können die Gewebeteile beidseitig des Risses T schon derart vorbehandelt worden sein, dass die Kanten der Gewebteile mit Hilfe einer arthroskopischen Raspel bzw. eines mechanischen Shavers bereits bearbeitet worden sind, um das perimeniskale Synovium zu einer Heilungsantwort zu stimulieren. Sodann können die Gewebeteile zusammengehalten werden, bis schliesslich mit Hilfe der Kanüle C der Gewebehalter 1 eingebracht und anschliessend fixiert wird, sodass der Riss T schliesslich - bei eingebrachtem Gewebehalter 1 - zusammengezogen wird nach Art einer Klammer, so dass der Riss T heilen kann.

In Fig. 5 ist ein weiteres Ausführungsbeispiel eines erfindungsgemässen Gewebehalters 1a gezeigt. Dieses Ausführungsbeispiel weist wie das Ausführungsbeispiel gemäss Fig. 1 ebenfalls erste und zweite Befestigungsmittel 2a und 3a auf, welche ihrerseits jeweils zwei Dorne 21a,22a bzw. 31a,32a aufweisen, die sich um den Verbindungssteg 4a herum erstrecken. Anders als bei dem zuvor beschriebenen Ausführungsbeispiel des Gewebehalters sind die Dorne hier jedoch im wesentlichen (d.h. ausgenommen sind der Ansatz am Verbindungssteg, der ein wenig dicker ausgebildet ist sowie das freie Ende, welches ein wenig spitz zuläuft) zylinderförmig ausgebildet. Sie verlaufen aber dennoch wendelförmig, und zwar in Richtung voneinander weg, wenn man die Dorne 21a,22a,31a,32a der ersten und zweiten Befestigungsmittel 2a und 3a relativ zueinenander betrachtet und sie in Richtung zu ihrem freien Ende hin verfolgt. Dadurch wird der Abstand b zwischen den nach innen weisenden Flächen der Dorne 21a,31a bzw. 22a,32a kleiner, wenn man vom freien Ende her betrachtet dem Verlauf der Dorne in Richtung zum Verbindungssteg 4a folgt. Auf diese Weise kann man ebenfalls beim Eindrehen die zuvor anhand des anderen Ausführungsbeispiels beschriebene Klammerwirkung erzielen. Das Eindrehen des Gewebehalters 1a kann analog verfolgen wie oben beschrieben, zu diesem Zweck ist ebenfalls ein Ansaztstück 5a bei dem beschriebenen Ausführungsbeispiel vorgesehen. Durch eine Umkehrung des wendelförmigen Verlaufs der Dorne 21a,22a bzw. 31a,32a auf die jeweiligen Dorne des anderen Befestigungsmittels zu (wieder in Richtung zum freien Ende des jeweiligen Dorns hin betrachtet), könnte man, so es erwünscht ist, eine Spreizwirkung erzielen, also ein Auseinanderziehen der Gewebteile.

## Patentansprüche

1. Gewebehalter (1,1a), welcher erste Befestigungsmittel (2,2a) umfasst, die mit einem Gewebeteil verbindbar sind, und zweite Befestigungsmittel (3,3a), die mit einem weiteren Gewebeteil verbindbar sind, und welcher Gewebehalter ferner Verbindungsmittel (4) umfasst, die die beiden Befestigungsmittel (2,2a;3,3a) und damit die Gewebeteile relativ zueinander halten, **dadurch gekennzeichnet, dass** die ersten und zweiten Befestigungsmittel (2,2a;3,3a) jeweils mindestens einen gekrümmten Dorn (21,22,21a,22a;31,32,31a,32a) umfassen, welcher sich um die Längsachse des Verbindungsmittels herum erstreckt.

2. Gewebehalter nach Anspruch 1, **dadurch gekennzeichnet, dass** die Verbindungsmittel, welche die Dorne (21,22,21a,22a;31,32,31a,32a) und damit die Gewebeteile relativ zueinander halten, einen in Richtung der Längsachse des Gewebehalters verlaufenden Verbindungssteg (4,4a) umfassen, und dass sich die Dorne (21,22,21a,22a;31,32,31a,32a) um diesen Verbindungssteg (4,4a) herum erstrecken.

3. Gewebehalter nach Anspruch 2, **dadurch gekennzeichnet, dass** der axiale Abstand (b) zwischen dem Dorn bzw. den Dornen (21,22,21a,22a) der ersten Befestigungsmittel (2,2a) und dem Dorn bzw. den Dornen (31,32,31a,32a) der zweiten Befestigungsmittel (3,3a) in Richtung zum freien Ende der Dorne hin zunimmt, also vom freien Ende der Dorne ausgehend zum Verbindungssteg (4,4a) hin abnimmt.

4. Gewebehalter nach einem der vorangehenden Ansprüche, **dadurch gekennzeichnet, dass** die Dorne (21,22,21a,22a;31,32,31a,32a) sich wendelförmig um die Längsachse des Gewebehalters bzw. um den Verbindungssteg (4,4a) herum erstrecken.

5. Gewebehalter nach Anspruch 4, **dadurch gekennzeichnet**, die axiale Dicke (A) eines Dorns (21,22,21a,22a;31,32,31a,32a) in Richtung zum freien Ende des Dorns hin abnimmt.

6. Gewebehalter nach Anspruch 4, **dadurch gekennzeichnet, dass** die Dorne (21a,22a;31a,32a) im wesentlichen zylindrisch ausgebildet sind.

7. Gewebehalter nach einem der Ansprüche 2 bis 6, **dadurch gekennzeichnet, dass** die ersten und zweiten Befestigungsmittel (2,2a;3,3a) jeweils zwei Dorne (21,22,21a,22a;31,32,31a,32a) umfassen, wobei die Dorne (21,22,21a,22a;31,32,31a,32a) des jeweiligen Befestigungsmittels (2,2a;3,3a) an gegenüberliegenden Orten von dem Verbindungssteg (4,4a) ausgehen und sich dann in gleicher Richtung um den Verbindungssteg (4,4a) herum erstrecken.

8. Gewebehalter nach einem der vorangehenden Ansprüche, **dadurch gekennzeichnet, dass** ein Ansatzstück (5,5a) für ein Werkzeug vorgesehen ist, mit welchem eine Verbindung des Gewebehalters an den Gewebeteilen bewirkt werden kann.

9. Gewebehalter (1,1a) nach einem der vorangehenden Ansprüche, **dadurch gekennzeichnet, dass** er aus einem bioresorbierbaren Material, insbesondere aus Polylactiden, hergestellt ist.

## Claims

1. Tissue holder (1, 1a), comprising first securing means (2, 2a) which can be connected to a tissue part and second securing means (3, 3a) which can be connected to a further tissue part, said tissue holder further comprising connection means which hold the two securing means (2, 2a; 3, 3a) and thus the tissue parts relative to one another, **characterized in that** the first and second securing means (2, 2a; 3, 3a) each comprise at least one curved spike (21, 22, 21a, 22a; 31, 32, 31a, 32a) which extends around the longitudinal axis of the connection means.

2. Tissue holder in accordance with claim 1, **characterized in that** the connection means which hold the spikes (21, 22, 21a, 22a; 31, 32, 31a, 32a) and thus the tissue parts relative to one another comprise a connecting web (4, 4a) which extends in the direction of the longitudinal axis of the tissue holder; and **in that** the spikes (21, 22, 21a, 22a; 31, 32, 31a, 32a) extend around this connecting web (4, 4a).

3. Tissue holder in accordance with claim 2, **characterized in that** the axial spacing (b) between the spike or the spikes (21, 22, 21a, 22a) of the first securing means (2, 2a) and the spike or the spikes (31, 32, 31a, 32a) of the second securing means (3, 3a) increases in the direction towards the free end of the spikes, that is decreases in the direction towards the connecting web (4, 4a) starting from the free end of the spikes.

4. Tissue holder in accordance with any one of the preceding claims, **characterized in that** the spikes (21, 22, 21a, 22a; 31, 32, 31a, 32a) extend helically around the longitudinal axis of the tissue holder or around the connecting web (4, 4a).

5. Tissue holder in accordance with claim 4, **characterized in that** the axial thickness (A) of a spike (21, 22, 21a, 22a; 31, 32, 31a, 32a) decreases in the direction towards the free end of the spike.

6. Tissue holder in accordance with claim 4, **characterized in that** the spikes (21a, 22a; 31a, 32a) are substantially cylindrical.

7. Tissue holder in accordance with any one of the claims 2 to 6, **characterized in that** the first and second securing means (2, 2a; 3, 3a) each comprise two spikes (21, 22, 21a, 22a; 31, 32, 31a, 32a), with the spikes (21, 22, 21a, 22a; 31, 32, 31a, 32a) of the respective securing means (2, 2a; 3, 3a) starting from the connecting web (4, 4a) at oppositely disposed positions and then extending in the same direction around the connecting web (4, 4a).

8. Tissue holder in accordance with any one of the preceding claims, **characterized in that** an extension piece (5, 5a) is provided for a tool, by means of which a connection of the tissue holder to the tissue parts can be effected.

9. Tissue holder (1, 1a) in accordance with any one of the preceding claims, **characterized in that** it is manufactured of a bioabsorbable material, in particular of polyactides.

## Revendications

1. Dispositif (1, 1a) de maintien de tissus, comprenant des premiers moyens de fixation (2, 2a) pouvant être reliés à une partie tissulaire, et des seconds moyens de fixation (3, 3a) pouvant être reliés à une autre partie tissulaire, ledit dispositif de maintien de tissus comportant, en outre, des moyens de liaison (4) qui assurent la retenue relative des deux moyens de fixation (2, 2a ; 3, 3a), et donc des parties tissulaires, **caractérisé par le fait que** les premiers et seconds moyens de fixation (2, 2a ; 3, 3a) englobent, respectivement, au moins un éperon courbe (21, 22, 21a, 22a ; 31, 32, 31a, 32a) s'étendant tout autour de l'étendue longitudinale du moyen de liaison.

2. Dispositif de maintien de tissus selon la revendication 1, **caractérisé en ce que** les moyens de liaison assurant la retenue relative des éperons (21, 22, 21a, 22a ; 31, 32, 31a, 32a), et donc des parties tissulaires, comprennent une membrure de solidarisation (4, 4a) s'étendant dans la direction de l'axe longitudinal dudit dispositif de maintien de tissus ; et par le fait que lesdits éperons (21, 22, 21a, 22a ; 31, 32, 31a, 32a) s'étendent tout autour de cette membrure de solidarisation (4, 4a).

3. Dispositif de maintien de tissus selon la revendication 2, **caractérisé en ce que** la distance axiale (b), respectivement comprise entre l'éperon ou les éperons (21, 22, 21a, 22a) des premiers moyens de fixation (2, 2a) et l'éperon ou les éperons (31, 32, 31a, 32a) des seconds moyens de fixation (3, 3a), croît en direction de l'extrémité libre desdits éperons, c'est-à-dire décroît vers la membrure de solidarisation (4, 4a) à partir de l'extrémité libre desdits éperons.

4. Dispositif de maintien de tissus selon l'une des revendications précédentes, **caractérisé en ce que** les éperons (21, 22, 21a, 22a ; 31, 32, 31a, 32a) s'étendent respectivement de manière hélicoïdale tout autour de l'axe longitudinal dudit dispositif de maintien de tissus, ou tout autour de la membrure de solidarisation (4, 4a).

5. Dispositif de maintien de tissus selon la revendication 4, **caractérisé en ce que** l'épaisseur axiale (A) d'un éperon (21, 22, 21a, 22a ; 31, 32, 31a, 32a) décroît en direction de l'extrémité libre dudit éperon.

6. Dispositif de maintien de tissus selon la revendication 4, **caractérisé en ce que** les éperons (21a, 22a ; 31a, 32a) sont de réalisation sensiblement cylindrique.

7. Dispositif de maintien de tissus selon l'une des revendications 2 à 6, **caractérisé en ce que** les premiers et seconds moyens de fixation (2, 2a ; 3, 3a) comprennent respectivement deux éperons (21, 22, 21a, 22a ; 31, 32, 31a, 32a), sachant que les éperons (21, 22, 21a, 22a ; 31, 32, 31a, 32a) du moyen de fixation respectif (2, 2a ; 3, 3a) partent de la membrure de solidarisation (4, 4a), en des emplacements opposés, puis s'étendent ensuite dans une même direction tout autour de ladite membrure de solidarisation (4, 4a).

8. Dispositif de maintien de tissus selon l'une des revendications précédentes, **caractérisé en ce qu'**il est prévu une pièce de rattachement (5, 5a) destinée à un outil permettant de provoquer une liaison dudit dispositif de maintien de tissus avec les parties tissulaires.

9. Dispositif (1, 1a) de maintien de tissus selon l'une des revendications précédentes, **caractérisé en ce qu'**il est fabriqué en un matériau biorésorbable, notamment en des polylactides.
